# EUROPÄISCHE PATENTSCHRIFT

(11) **EP 2 997 989 B1**
(45) Veröffentlichungstag und Bekanntmachung des Hinweises auf die Patenterteilung: **03.05.2017**
(21) Anmeldenummer: 15002205.1
(22) Anmeldetag: 25.07.2015
(51) Int. Cl.: A61M 5/162, A61M 1/32

(54) **VORRICHTUNG ZUR OZON-EIGENBLUT-INFUSION**
DEVICE FOR OZONE AUTOLOGOUS BLOOD INFUSION
DISPOSITIF DE PERFUSION OZONE-SANG AUTOLOGUE

(30) Priorität: 08.09.2014 DE 102014012969
(43) Veröffentlichungstag der Anmeldung: 23.03.2016
(73) Patentinhaber: Dr. J. Hänsler GmbH, 76473 Iffezheim (DE)
(72) Erfinder: Viebahn-Hänsler, Renate, 76473 Iffezheim (DE)
(74) Vertreter: Flosdorff, Jürgen

(56) Entgegenhaltungen:
- EP-A2- 0 385 320
- WO-A1-2008/067506
- WO-A2-2006/125212
- CN-U- 203 577 022
- DE-A1- 3 109 691
- GB-A- 1 257 419
- US-A- 2 409 343
- US-A1- 2012 226 236

## Beschreibung

Die Erfindung betrifft eine Vorrichtung zur Ozon-Eigenblut-Infusion, mit einer Vakuumflasche mit einem Stopfen, der den Flaschenhals der Vakuumflasche luftdicht verschließt, mit einem Transfusionsbesteck und einem Keimstopp, die jeweils einen Zuführungsschlauch aufweisen, und mit einem Gaszuführungsröhrchen in der Vakuumflasche, das in dem Stopfen gehalten ist. Eine derartige Vorrichtung zur sogenannten Großen Ozon-Eigenblut-Behandlung dient der Re-Infusion von ozonbehandeltem Eigenblut in einem geschlossenen, sterilem System. Dabei werden 50 bis 100 mml Eigenblut in einer evakuierten Flasche, die üblicherweise als "Plasmaflasche" bezeichnet wird, mit einem Ozon-Sauerstoffgemisch versetzt und das Reaktionsprodukt als Tropfinfusion reinfundiert.

In der mit einem Stopfen dicht verschlossenen, evakuierten "Plasmaflasche" befindet sich ein Gaszuführungsröhrchen, das mit seinem oberen Endbereich in dem Stopfen befestigt ist, ohne dabei den Stopfen ganz zu durchdringen. Das Gaszuführungsröhren erstreckt sich bis in die Nähe des Bodens der "Plasmaflasche" und ist im unteren Endbereich mit einem sogenannten Mikroperlsystem versehen, mit dem das in das Gaszuführungsröhrchen eingebrachte Ozon-Sauerstoff-Gemisch in das in der Flasche stehende Blut in kleinen Gasbläschen verteilt eintritt, um so eine gleichmäßige Verteilung der wirksamen Komponente 03 im Blut zu erreichen.

Die kurz als "Keimstopp" bezeichnete Keimstopp-Ozon-Perfusionsleitung enthält einen Bakterienfilter und eine Rollklemme. Der Keimstopp kann außerdem mit einer Einmalspritze verbunden werden und dient dazu, das Ozon-Sauerstoff-Gasgemisch in die Vakuumflasche einzuführen. Das Ozon-Sauerstoff-Gemisch wird in einem Ozongenerator erzeugt. Wenn das benötigte Ozon-Sauerstoff-Gemisch in die Einmalspritze des Keimstopps aufgenommen wird, kann die Vorrichtung an einem von dem Ozongenerator entfernten Ort benutzt werden. Der Keimstopp wird an der Stelle, an der das Gaszuführungsröhrchen im unteren Bereich des Stopfens befestigt ist, in den Stopfen eingeführt, woraufhin das Ozon-Sauerstoff-Gemisch durch das Vakuum in der "Plasmaflasche" eingesaugt wird.

Zuvor ist Eigenblut des Benutzers durch das Transfusionsbesteck, das ebenfalls mit einer Rollklemme versehen ist, in die "Plasmaflasche" eingesaugt worden, nachdem der Zuführungsschlauch des Transfusionsbestecks an der dafür vorgesehenen Stelle in den Flaschenstopfen eingestochen wurde. Nach Durchmischung des Bluts mit dem im Blut fein verteilten Ozon-Sauerstoff-Gemisch kann das Blut in den Benutzer reinfundiert werden.

Gegenwärtig werden der Keimstopp mit Hilfe einer Kanüle und das Transfusionsbesteck mittels eines Einstechdorns durch den Stopfen der Vakuumflasche gestochen. Da die Vakuumflaschen bisher mit Latex-haltigen Stopfen verschlossen sind, werden der Einstechdorn und die Kanüle dicht umschlossen, wobei bei Entfernen des Einstechdorns und/oder der Kanüle das entstandene Loch wieder dicht verschlossen wird, so dass das Vakuum in der Flasche erhalten bleibt. Dies ist für den Gebrauch der Vorrichtung äußerst wichtig. Allerdings hat die Verwendung von Latex(Naturkautschuk) bei den Stopfen den Nachteil, dass bei Patienten heftige allergische Reaktionen auftreten können, weil bei der Reinfusion das Patientenblut etwa 10 min mit dem Stopfen in Kontakt kommt.

Die Verwendung Latex-freier Stopfen bringt bisher Probleme mit sich, weil die Plastizität schlechter und ihre mechanische Belastbarkeit geringer ist und die Dichtigkeit der Stopfen allenfalls bei sorgfältigster Handhabung aufrecht zu erhalten wäre. Dies bedeutet, dass das Einstechen des Keimstopps und des Transfusionsbestecks auf herkömmliche Weise nicht korrigiert werden könnte, da sich der Stopfen nicht wieder vollständig schließen und das Vakuum der Flasche damit gefährdet wäre. Wenn zudem die Kanüle des Keimstopps nicht vollständig eingeführt wird, kollidiert dieser mit dem Einstechdorn oder der dickbauchigen Filterkammer des herkömmlichen Transfusionsbestecks, wobei dann Scherkräfte an der Kanüle auftreten, die das Einstechloch dehnen, das sich nicht wieder zurückbilden kann, so dass auch aus diesem Grund das Vakuum der Flasche verloren geht. Wenn zudem das Transfusionsbesteck zügig eingeführt wird, besteht die Gefahr der Stopfenverletzung durch rauhe, teilweise scharfe Kunststoffteile am Übergang des Einstechdorns zur Filterkammer des Transfusionsbestecks. Auch beim kräftigen Einstechen der Keimstopp-Kanüle kann der Stopfen durch den Kanülen-Ansatz (gespritzte Kunststoffteile) mechanisch verletzt werden. Die obigen Punkte könnten somit zum Verlust des Vakuums der Flaschen führen und eine normale Handhabung der herkömmlichen Vorrichtung bei Verwendung eines Latex-freien Stopfens erschweren oder verhindern. Auch eine sorgfältige Handhabung unter Berücksichtigung der obigen Punkte könnte das Problem nur teilweise beheben.

Die US 2012/226236 A1 offenbart einen Medikamentenapplikationsadapter mit Gassperrelement für ein Hämodialyse-Schlauchset. Dabei ist eine Medikamentenlösung in eine handelsübliche Durchsteckflasche aufgenommen, deren Stopfen von einem doppellumigen Spike durchstoßen wird, der mit zwei Leitungswegen verbunden ist. Der doppellumige Spike ist an einer Aufnahmevorrichtung befestigt, die die Durchstechflasche mit der Öffnung nach unten in ihrer Position hält. Zu diesem Zweck sind an der Aufnahmevorrichtung Rastnasen vorgesehen, die hinter dem Hals der Durchstechflasche einrasten, um diese gegen ein ungewolltes Abkoppeln abzusichern. Implizit ist offenbart, dass der Stopfen aus einem Latex-freien Material besteht, weil dies bei der Infusion von Medikamenten allgemein üblich Dieses Dokument zeigt damit eine Vorrichtung gemäss dem Oberbegriff des Anspruchs 1.

Die US 2,409,343 A, WO2008/067506 A1 und GB 1 257 419 A offenbaren doppellumige Einstechnadeln.

Die EP 0 385 320 A2 offenbart ein Gaszuführungsröhrchen in einer Vakuumflasche für die Ozonisierung von Blut für die Eigenbluttherapie.

Der vorliegenden Erfindung liegt die Aufgabe zugrunde, eine Vorrichtung der betrachteten Art so zu verbessern, dass sowohl die oben erwähnten heftigen allergischen Reaktionen von Patienten als auch ein Verlust des Vakuums in den "Plasma-Flaschen" vermieden sind.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht einen Adapter vor, an dem zwei Kanülen so befestigt sind, dass ihre Spitzen gemeinsam in den Stopfen einstechbar sind, wobei an ihren gegenüber liegenden Enden die Zuführungsschläuche des Transfusionsbestecks und des 4 Keimstopps befestigbar sind. Mit Hilfe dieses Adapters läßt sich das Einstechen des Stopfens so korrekt durchführen, dass eine Korrektur nicht erforderlich ist. Dabei ist erfindungsgemäß vorgesehen, dass der Stopfen kein Latex enthält, so dass keine allergische Reaktion bei der Re-Infusion bei dem Patienten vorkommen kann. Der Stopfen besteht vorzugsweise aus synthetischem Kautschuk.

Der Stopfen enthält erfindungsgemäß Markierungen zum Einstechen der Kanülen, die an solchen Stellen der Bodenwand des Adapters befestigt sind, dass die Kanüle mit den Markierungen fluchten können. Der Adapter besteht aus einem durchsichtigen Kunststoff, so dass beim Ansetzen des Adapters an dem Stopfen die Markierungen zum Einstechvorgang exakt getroffen werden können. Geeignete Materialien für den Adapter sind beispielsweise durchsichtiges PC oder PVC, ohne dass die Erfindung hierauf beschränkt ist.

Nach einem weiteren Vorschlag der Erfindung hat der Adapter im wesentlichen eine Kappen- oder Napf-Form. Er hat eine Bodenwand, durch die die beiden Kanülen im wesentlichen im rechten Winkel zu dieser verlaufen, wobei die Kanülen in die Bodenwand eingeschweißt, eingeklebt oder eingeclipst sind, so dass sie starr an der Bodenwand befestigt sind. Der Adapter sollte außerdem eine mit der Bodenwand einstückige zylindrische Umfangswand aufweisen, die beim Gebrauch des Adapters den Flaschenhals oder eine Umbördelung des Flaschenhalses vorzugsweise mit geringem Spiel umgreift.

Die Kanülen ragen mit ihren rückseitigen Endbereichen aus der Oberseite der Bodenwand des Adapters heraus und sind dort zweckmäßigerweise mit Führungsröhrchen verbunden, auf die die Zuführschläuche des Keimstopps und des Transfusionsbestecks aufsteckbar sind.

Außerdem wird mit Vorteil vorgeschlagen, dass die innere Bodenwand des Adapters einen vorzugsweise ringförmigen, radial äußeren Vorsprung aufweist, der auf der Oberseite des Stopfens aufliegen kann, wenn der Adapter an dem Flaschenhals befestigt ist. In diesem Zusammenhang wird vorgeschlagen, dass der Flaschenhals eine vorzugsweise aus Metall bestehende Umbördelung aufweist, und dass innen am Randbereich der Umfangswand des Adapters ein Clips-Ring oder gegenüber liegende Clips-Vorsprünge angeformt sind, die unter der Umbördelung einrasten können. Hierdurch ist der Adapter sicher an der "Plasma-Flasche" befestigt, was auch für die Überkopf-Lage der Flasche bei der Re-Infusion des Blutes wichtig ist.

Die Zuführungsschläuche des Keimstopps und des Transfusionsbestecks sind zweckmäßigerweise in einem doppelseitigen Führungsclip dicht oberhalb des Adapters gehalten, wobei der Führungsclip zum Einlegen der Schläuche seitlich geschlitzt sein kann.

Die Erfindung wird nachfolgend mit Bezug auf die Zeichnungen näher erläutert. Dabei zeigen:

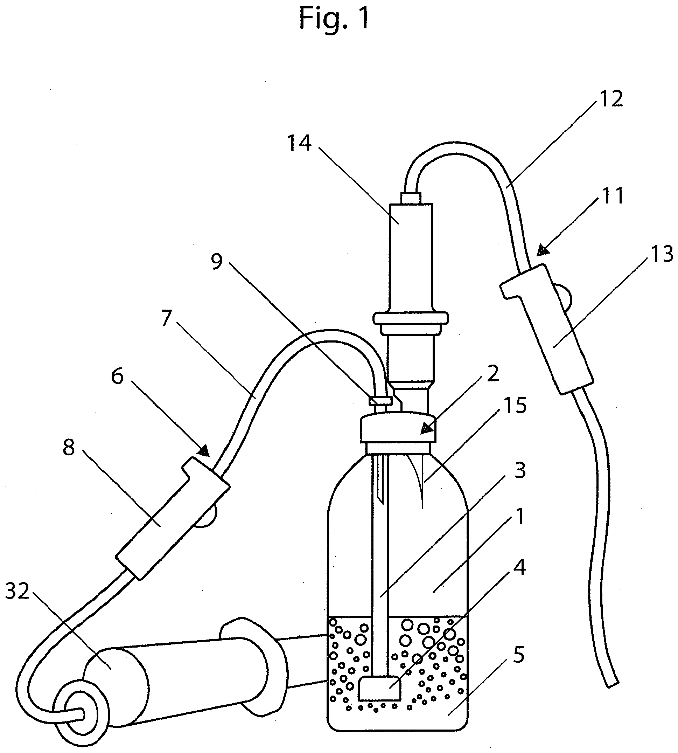

- Figur 1: eine herkömmliche Vorrichtung zur Ozon-Eigenblut-Infusion;
- Figur 2: eine Aufsicht auf den Stopfen der Vorrichtung gemäß Figur 1;
- Figur 3: eine Unteransicht eines erfindungsgemäß vorgesehenen Adapters mit eingeschweißten Kanülen;
- Figur 4: der Adapter der Figur 3 ohne Kanülen;
- Figur 5: das Einführen der Kanülen in Markierungen eines Flaschenstopfens;
- Figur 6: den Adapter mit Führungs-Clips für die Zuführschläuche;
- Figur 7: die Führungs-Clips der Figur 6;
- Figuren 8A, 8B und 8C: eine Unteransicht, eine Schnittdarstellung und eine Aufsicht des Adapters.

Eine herkömmliche Vorrichtung zur Ozon-Eigenblut-Infusion enthält eine Vakuumflasche 1, die durch einen Stopfen 2 luftdicht verschlossen ist. In einem unteren Bereich des Stopfens 2 ist ein Gaszuführungsröhrchen 3 befestigt, das den Stopfen 2 nicht vollständig durchdringt und an seinem unteren Ende mit einem Mikro-Perl-System 4 verbunden ist, das ein zugeführtes O₂/O₃-Gas-Gemisch in dem in der Flasche 1 befindlichen Blut 5 fein verteilt.

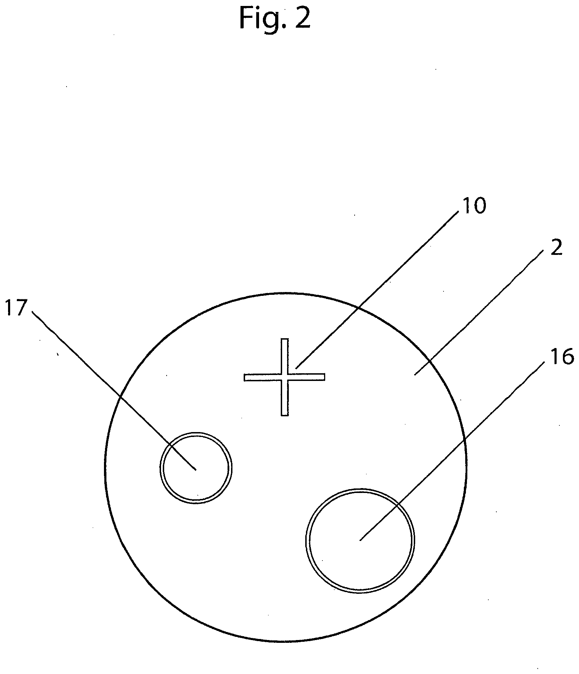

Eine insgesamt als "Keimstopp" 6 bezeichnete Einrichtung enthält einen Zuführungsschlauch 7, der mit einer Rollklemme 8 versehen ist und an einem Ende eine Kanüle 9 enthält, die an der Stelle in den Stopfen 2 eingestochen wird, die mit dem Gaszuführungsröhrchen 3 fluchtet und in Figur 2 mit einem Kreuzsymbol 10 bezeichnet ist. Das andere Ende des Zuführungsschlauchs 7 ist mit einer vorzugsweise 50 ml großen Einmalspritze 32 verbindbar, die mit dem O₂/O₃-GasGemisch gefüllt werden kann, das nach dem Einstechen durch das Vakuum in der Flasche 1 eingesaugt wird.

Ein insgesamt mit dem Bezugszeichen 11 bezeichnetes Transfusionsbesteck enthält einen Zuführungsschlauch 12, der ebenfalls mit einer Rollklemme 13 versehen ist und eine Filterkammer 14 und einen anschließenden Einstechdorn 15 aufweist. Der Zugang für das Transfusionsbesteck 11 ist in Figur 2 durch den großen Kreis 16 markiert. Der kleine Kreis 17 in der Figur 2 kennzeichnet eine zusätzliche Belüftungsmöglichkeit, und ist auch als Einstichstelle zur evtl. Zugabe von Medikamenten vorgesehen.

Durch das Transfusionsbesteck 11 wird das mit Ozon angereicherte Eigenblut als Tropfinfusion reinfundiert.

Der Stopfen 2 der herkömmlichen Vorrichtung enthält Latex, das - wie oben erwähnt - allergische Reaktionen bei Patienten hervorrufen kann.

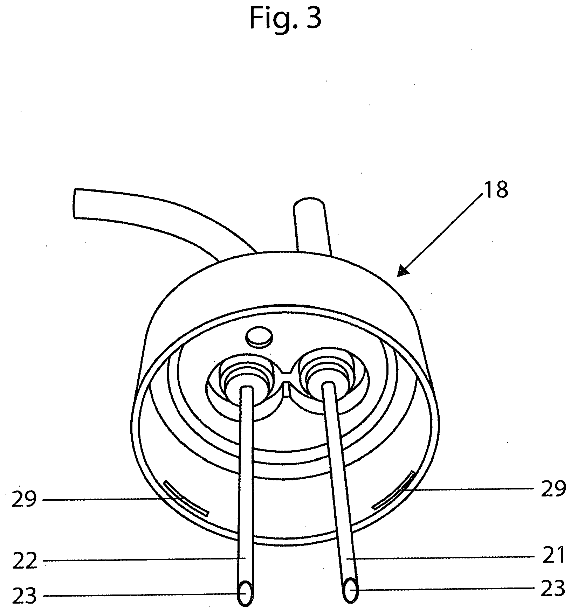

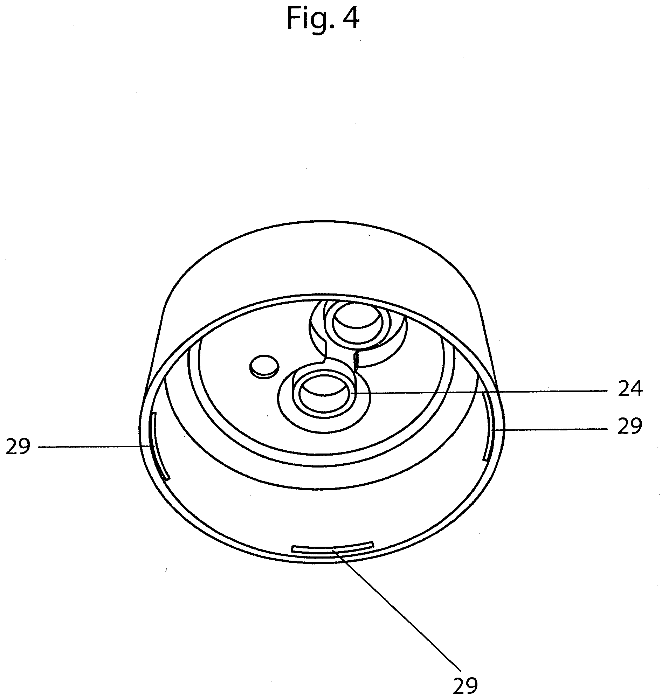

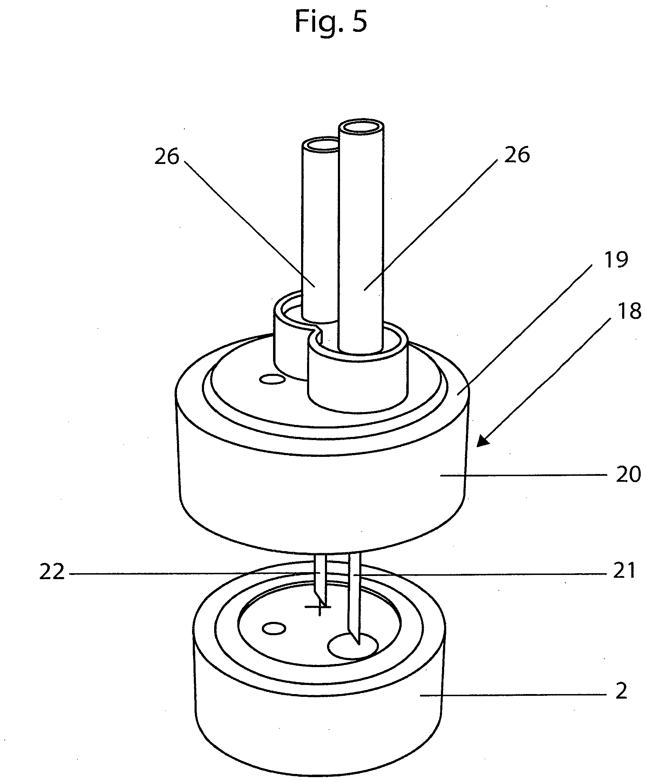

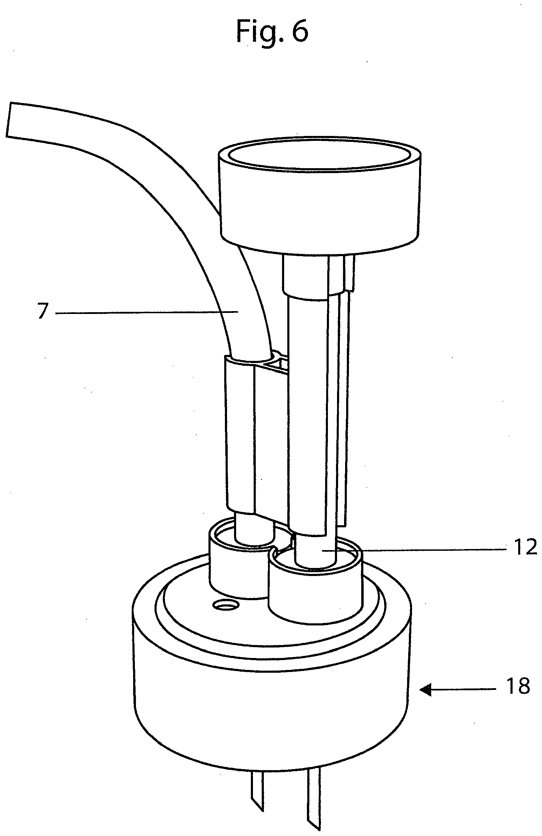

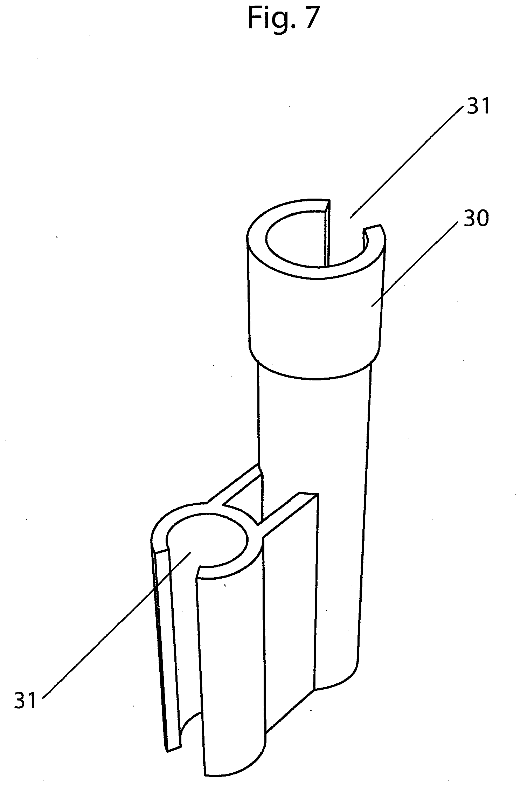

Die Figuren 3 bis 8 zeigen eine bevorzugte Ausführungsform der vorliegenden Erfindung. Diese sieht einen Adapter 18 vor, der insgesamt eine etwa kreiszylindrische Form hat und aus einem durchsichtigen Kunststoff besteht. Der Adapter 18 enthält eine Bodenwand 19 und eine kreiszylindrische Umfangswand 20. In die Bodenwand 19 sind zwei Kanülen 21, 22 eingeschweißt, eingeklebt oder eingeclipst, so dass sie in der Bodenwand 19 unbeweglich fixiert sind. Die Kanülen 21, 22 sind an solchen Stellen in dem Adapter 18 befestigt, dass sie beim Aufsetzen des Adapters 18 auf die Flasche 1 mit den Markierungen 10, 16 des Stopfens 2 fluchten können. Die Kanülen 21, 22, deren Spitzen 23 gleich weit von dem Adapter 18 vorstehen, werden gleichzeitig in den Stopfen 2 eingestochen, wobei die Kanüle des Keimstopps 6 ins Innere des Gaszuführungsröhrchens 3 hineinragt. Die andere Kanüle ragt ein paar Millimeter ins Innere der Flasche hinein.

Zur stabilen Befestigung der Kanülen 21, 22 in Längsrichtung des Adapters 18 und der Flasche 1 sind an den Befestigungsstellen zwei rohrförmige Ansätze 24 an dem etwa stufenförmigen Boden 19 des Adapters 18 ausgebildet. In diesen rohrförmigen Ansätzen 24 sind die beiden Kanülen 21, 22 auf die oben genannte Weise starr befestigt. Während die kleineren rohrförmigen Ansätze 24 ins Innere des Adapters 18 ragen, stehen von der Oberseite des Bodens 19 rohrförmige Ansätze 25 eines größeren Durchmessers ab. In diesem Bereich sind auf die Kanülen 21, 22 Führungsröhrchen 26 aufgesetzt, auf die ihrerseits die Schläuche 7, 12 aufgesetzt werden. Bei der erfindungsgemäßen Vorrichtung besteht das Transfusionsbesteck 11 aus einem Kanülen-Schlauch-Filterkammer-System.

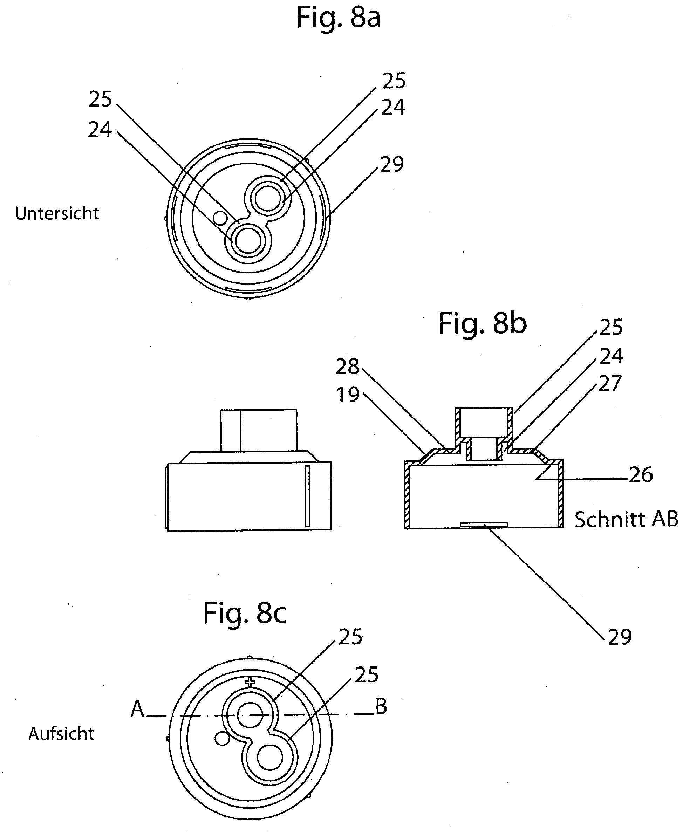

Wie insbesondere Figur 8B zeigt, hat der Boden 19 des Adapters 18 einen radial äußeren Ringbereich 26, mit dem der Adapter 18 in dem an der Flasche 1 befestigten Zustand auf dem Flaschenhals bzw. einer den Flaschenhals und den Rand des Stopfens umgreifenden Umbördelung aufliegt. Im Anschluss an den Ringbereich 26 geht ein konischer Ringbereich 27 in einen ebenen inneren Ringbereich 28 über, an den sich die rohrförmigen Ansätze 25 anschließen. Die kleineren zylindrischen Ansätze 24, in denen die Kanülen 21, 22 befestigt sind, enden oberhalb der Auflagefläche 26, so dass sie nicht mit dem Stopfen in Berührung kommen.

Am Rand der Innenseite der Umfangswand 20 des Adapters 18 sind Stege 29 angeformt, die unter einer nicht dargestellten Umbördelung des Flaschenhalses clips-artig einrasten.

Oberhalb des Adapters 18 ist ein doppelseitiger Führungs-Clip 30 aus einem verhältnismäßig starren Material vorgesehen, der die Schläuche 7, 12 im Verbindungsbereich mit dem Adapter 18 über seine Länge in vertikaler Ausrichtung hält. Der Führungs-Clip 30 hat seitliche Schlitze 31 zum Eintritt der Schläuche 7, 12.

## Patentansprüche

1. Vorrichtung zur Ozon-Eigenblut-Infusion, mit einer Vakuumflasche (1) mit einem Stopfen (2), der den Flaschenhals der Vakuumflasche (1) luftdicht verschließt, mit einem Transfusionsbesteck (11) und einem Keimstopp (6), die jeweils einen Zuführungsschlauch (12,7) aufweisen, ferner mit einem Adapter (18) mit zwei daran befestigten Kanülen (21,22), deren Spitzen (23) gemeinsam in den Stopfen (2) einstechbar sind und an deren gegenüberliegenden Enden die Zuführungsschläuche (12,7) befestigbar sind, wobei der Stopfen (2) aus einem Latex-freien Material besteht,
**dadurch gekennzeichnet,**
**dass** ein Gaszuführungsröhrchen (3) in der Vakuumflasche angeordnet ist, das in dem Stopfen (2) gehalten ist,
**dass** der Stopfen (2) Markierungen (10,16) zum Einstechen der Kanülen (21,22), wobei eine (10) der Markierungen die Stelle des Stopfens markiert, die mit dem Gaszuführungsröhrchen (3) fluchtet, und die Kanülen (21, 22) an solchen Stellen des Adapters (18) befestigt sind, dass die Kanülen (21, 22) mit den Markierungen (10,16) fluchten können, und dass der Adapter (18) aus einem durchsichtigen Kunststoff besteht.

2. Vorrichtung nach Anspruch 1,
**dadurch gekennzeichnet,**
**dass** der Stopfen (2) aus synthetischem Kautschuk besteht.

3. Vorrichtung nach Anspruch 1 oder 2,
**dadurch gekennzeichnet,**
**dass** der Adapter (18) eine Bodenwand (19) aufweist, durch die die beiden Kanülen (21,22) im wesentlichen im rechten Winkel zur Ebene der Bodenwand (19) verlaufen, wobei die Kanülen (21,22) in die Bodenwand (19) eingeschweißt, eingeklebt oder eingeklipst sind.

4. Vorrichtung nach einem der Ansprüche 1 bis 3,
**dadurch gekennzeichnet,**
**dass** der Adapter (18) außerdem eine mit der Bodenwand (19) einstückige zylindrische Umfangswand (20) aufweist, die den Flaschenhals oder eine Umbördelung des Flaschenhalses umgreift.

5. Vorrichtung nach einem der Ansprüche 1 bis 4,
**dadurch gekennzeichnet,**
**dass** die Kanülen (21,22) über den Befestigungsstellen an dem Adapter (18) mit Führungsröhrchen (26) versehen sind, auf die die Zuführschläuche (12,7) aufsteckbar sind.

6. Vorrichtung nach einem der Ansprüche 1 bis 5,
**dadurch gekennzeichnet,**
**dass** die innere Bodenwand des Adapters (18) einen vorzugsweise ringförmigen, radial äußeren Vorsprung (26) aufweist, der auf der Oberseite des Stopfens (2) aufliegen kann.

7. Vorrichtung nach einem der Ansprüche 1 bis 6,
**dadurch gekennzeichnet,**
**dass** der Flaschenhals eine vorzugsweise aus Metall bestehende Umbördelung aufweist und dass innen am Randbereich der Umfangswand des Adapters (18) ein Clips-Ring oder gegenüberliegende Clips-Vorsprünge (29) angeformt sind, die unter der Umbördelung einrasten können.

8. Vorrichtung nach einem der Ansprüche 1 bis 7,
**dadurch gekennzeichnet,**
**dass** die Zuführungsschläuche (7,12) in einem doppelseitigen Führungsclip (30) dicht oberhalb des Adapters (18) gehalten sind.

## Claims

1. Apparatus for ozone-autologous blood infusion including a vacuum bottle (1) with a stopper (2), which closes the throat of the vacuum bottle (1) in an airtight manner, a transfusion instrument (11) and a germ stop (6), which each have a supply hose (12, 7), and also an adapter (18) with two cannulas (21, 22) attached thereto, the tips (23) of which may be pushed together into the stopper (2) and to whose opposite ends the supply hoses (12, 7) may be fastened, wherein the stopper (2) consists of a latex-free material, **characterised in that** a gas supply tube (3) is arranged in the vacuum bottle, which is held in the stopper (2), that the stopper (2) has markings (10, 16) for the penetration of the cannulas (21, 22), wherein one (10) of the markings marks the position on the stopper, which is in alignment with the gas supply tube (3) and the cannulas (21, 22) are fastened at such positions on the adapter (18) that the cannulas (21, 22) can be aligned with the markings (10, 16) and that the adapter (18) consists of a transparent plastic material.

2. Apparatus as claimed in Claim 1, **characterised in that** the stopper (2) consists of synthetic rubber.

3. Apparatus as claimed in Claim 1 or 2, **characterised in that** the adapter (18) has a base wall (19), through which the two cannulas (21, 22) extend substantially at right angles to the plane of the base wall (19), wherein the cannulas (21, 22) are welded, secured by adhesive or clipped into the base wall (19).

4. Apparatus as claimed in one of Claims 1 to 3, **characterised in that** the adapter (18) also has a cylindrical peripheral wall (20), which is integral with the base wall (19) and engages around the bottle throat or a bead on the bottle throat.

5. Apparatus as claimed in one of Claims 1 to 4, **characterised in that** the cannulas (21, 22) are provided above the fastening points to the adapter (18) with guide tubes (26), onto which the supply hoses (12, 17) may be pushed.

6. Apparatus as claimed in one of Claims 1 to 5, **characterised in that** the inner base wall of the adapter (18) has a preferably annular, radially outer projection (26), which can engage the upper surface of the stopper (2).

7. Apparatus as claimed in one of Claims 1 to 6, **characterised in that** the bottle throat has a bead which preferably consists of metal and integrally formed internally in the edge region of the peripheral wall of the adapter (18) there is a clip ring or opposing clip projections (20), which can lock beneath the bead.

8. Apparatus as claimed in Claims 1 to 7, **characterised in that** the supply hoses (7, 12) are retained in a double-sided guide clip (30) closely above the adapter (18).

## Revendications

1. Dispositif pour la perfusion d'ozone-sang autologue, comportant une bouteille à vide (1) dotée d'un bouchon (2) qui ferme de façon étanche à l'air le col de la bouteille à vide (1), d'un instrument de transfusion (11) et d'un système anti-germe (6) qui présentent respectivement un tuyau d'acheminement (12, 7), doté en outre d'un adaptateur (18) avec deux canules fixées à celui-ci (21, 22) dont les pointes (23) peuvent être insérées dans le bouchon (2) et sur leurs extrémités opposées, les tuyaux d'acheminement (12, 7) peuvent être fixés, dans lequel le bouchon (2) consiste en un matériau sans latex,
**caractérisé en ce que**
un tube d'acheminement de gaz (3) est disposé dans la bouteille à vide qui est contenu dans le bouchon (2),
le bouchon (2) comporte des marques (10, 16) pour l'insertion des canules (21, 22), dans lequel l'une (10) des marques marque l'emplacement du bouchon qui est aligné avec le tube d'acheminement de gaz (3),
et les canules (21, 22) sont fixées sur cet emplacement de l'adaptateur (18), **en ce que** les canules (21, 22) peuvent être alignées avec les marques (10, 16), et
**en ce que** l'adaptateur (18) consiste en une matière plastique transparente.

2. Dispositif selon la revendication 1,
**caractérisé en ce que**
le bouchon (2) consiste en caoutchouc synthétique.

3. Dispositif selon la revendication 1 ou 2,
**caractérisé en ce que**
l'adaptateur (18) présente une paroi inférieure (19) à travers laquelle passent les deux canules (21, 22) essentiellement à angle droit par rapport au plan de la paroi inférieure (19), dans lequel les canules (21, 22) sont soudées, collées ou clipsées dans la paroi inférieure (19).

4. Dispositif selon l'une des revendications 1 à 3,
**caractérisé en ce que**
l'adaptateur (18) présente en outre une paroi périphérique (20) cylindrique d'un seul tenant avec la paroi inférieure (19) qui entoure le col de la bouteille ou un bord rabattu du col de la bouteille.

5. Dispositif selon l'une des revendications 1 à 4,
**caractérisé en ce que**
les canules (21, 22) sont dotées sur les emplacements de fixation sur l'adaptateur (18) de tubes d'acheminement (26) sur lesquels les tuyaux d'acheminement (12, 7) peuvent être fichés.

6. Dispositif selon l'une des revendications 1 à 5,
**caractérisé en ce que**
la paroi inférieure intérieure de l'adaptateur (18) présente une proéminence (26) extérieure radiale, de préférence annulaire qui peut reposer sur la partie supérieure du bouchon (2).

7. Dispositif selon l'une des revendications 1 à 6,
**caractérisé en ce que**
le col de la bouteille présente un bord rabattu de préférence en métal et **en ce que**, à l'intérieur sur la zone du pourtour de la paroi périphérique de l'adaptateur (18), un anneau de type clip ou des proéminences opposées de type clips (29) sont formés, qui peuvent s'encliqueter sous le bord rabattu.

8. Dispositif selon l'une des revendications 1 à 7,
**caractérisé en ce que**
les tuyaux d'acheminement (7, 12) sont maintenus de façon étanche au-dessus de l'adaptateur (18) dans un clip de guidage à deux côtés (30).
